# EUROPEAN PATENT SPECIFICATION

(11) **EP 1 747 258 B1**
(45) Date of publication and mention of the grant of the patent: **09.07.2008**
(21) Application number: 05787019.8
(22) Date of filing: 13.05.2005
(51) Int. Cl.: C11D 1/00

(54) **CLEANSING WIPES HAVING A COVALENTLY BOUND OLEOPHILIC COATING, THEIR USE AND PROCESSES FOR THEIR MANUFACTURE**
REINIGUNGSTÜCHER MIT EINER KOVALENT GEBUNDENEN OLEOPHILEN BESCHICHTUNG, IHRE VERWENDUNG UND VERFAHREN ZU IHRER HERSTELLUNG
AMELIORATIONS DE PRODUITS OU ASSOCIEES A CES PRODUITS

(30) Priority: 14.05.2004 GB 0410807
(43) Date of publication of application: 31.01.2007
(73) Proprietor: Reckitt Benckiser (UK) Limited, Slough, Berkshire SL1 3UH (GB)
(72) Inventor: DUFFIELD,P.J. Reckitt Benckiser Corp.ServicesLtd., Hull HU8 7DS (GB); GOODWIN, Andrew, James, County Cork (IE); LEADLEY, Stuart, Robert, County Cork (IE); McKECHNIE,M.T. Reckitt Benckiser Corp.Services Ltd, Hull HU8 7DS (GB); O'NEILL, Liam, County Tipperary (IE); PUGH, S. Reckitt Benckiser Corporate Services Ltd., Hull HU8 7DS (GB)
(74) Representative: Bowers, Craig Malcolm
(86) International application number: PCT/GB2005/001832
(87) International publication number: WO 2005/123891

(56) References cited:
- WO-A-00/16913
- WO-A-00/16914
- WO-A-03/086031
- WO-A-03/101621
- WO-A-20/05065603
- PATENT ABSTRACTS OF JAPAN vol. 014, no. 176 (C-0707), 9 April 1990 (1990-04-09) & JP 02 023922 A (TORAY IND INC), 26 January 1990 (1990-01-26)

## Description

The present invention relates to a process for incorporating active materials (hereafter referred to as "actives" or "active materials") in coating compositions obtained through plasma polymerisation or plasma enhanced chemical vapour deposition (PE-CVD).

The term "actives" or "active materials" as used herein is intended to mean materials that perform one or more specific functions when present in a certain environment and in the case of the present application are chemical species which do not undergo chemical bond forming reactions within a plasma environment. It is to be appreciated that an Active is clearly discriminated from the term "Reactive". A reactive chemical species is intended to mean a species which undergoes chemical bond forming reactions within a plasma environment. The active may, of course, be capable of undergoing a reaction after the coating process.

Actives are often present in formulated products in low concentrations and yet are typically the most costly component in the formulated product. For example, the UV absorbing or refracting component of a sun block emulsion formulated product or the decongestant and/or analgesic in a cold cure formulated product. Ensuring effective delivery of the active to the point of end application is a key requirement for good efficacy of the product.

Actives often need to be protected during processing and prior to end use in order that they are safely released and or activated or the like at the intended point of end use for both effective performance and effective cost. This is often achieved by incorporating the active into a protective matrix, applying a protective coating, or introducing the active into a matrix in a chemically protected form (i.e. the presence of protective end groups which will react with another species in the end use environment to release the active). The two former protective methods may be referred to in general terms as forms of encapsulation. For example many pharmaceutical materials are susceptible to acidic degradation and need to be protected from the acidic stomach prior to effective release and adsorption in the more alkaline intestine. In this case the encapsulating coatings are known as enteric coatings. Other additives must be protected from heat, moisture, or extremes of pH during processing as part of incorporation into the product matrix.

As well as protecting an active prior to and/or during delivery the encapsulating coating or matrix may also serve as a mechanism to control release of the active. This controlled release or sustained release ensures a controlled dosage of the active for a prolonged period of time. Controlled release is typically a diffusion-controlled process where the active diffuses through the encapsulating matrix or coating or the encapsulating material gradually dissolves in the environment in which the active is to be released.

Polymer matrices and polymeric coatings are often used as media for encapsulation and controlled release. A wide range of polymeric materials has been used for this purpose from natural macromolecules such as cellulose through to synthetic polymers such as polymers of methacrylic acid and methacrylate such as the EUDRAGIT^{®} range of products for enteric coatings from Degussa. In the case of coatings, these are often applied from solvent using traditional coating processes.

Polymeric coatings are widely used throughout industry because they are easily applied, to give conformal, filmic coatings on a wide range of substrates. The functionality of the polymer, for example, oil repellency, water barrier, biocompatibility, decorative, adhesive, release etc. is often provided to the substrate coated. An extensive range of methods are used for the delivery and/or curing of films or the like made from the polymeric coatings. As an example a polymer melt or solution is typically applied by mechanical coating or immersion of a substrate with the resulting polymeric coating being converted to a film by a suitable curing technique such as for example by the application of heat, radiation and/or pressure. More recently it has been demonstrated that thin, conformal polymeric films can be applied/deposited on substrates by means of plasma polymerisation or plasma enhanced chemical vapour deposition (PE-CVD) processes.

Conformal polymer films can be applied via the process of plasma polymerisation or plasma enhanced chemical vapour deposition (PE-CVD). Chemical Vapour Deposition is the deposition of a solid on a heated substrate from a chemical reaction in the vapour phase near or on the heated substrate. The chemical reactions which take place may include thermal decomposition, oxidation, carburisation and nitridation. Typically the sequence of events for a CVD reaction comprises the following sequentially:-
i) Introduction of reactant gases into a reactor by appropriate introduction means e.g. forced flow,
ii) diffusion of the gases through the reactor towards a substrate surface
iii) contact of gases with substrate surface
iv) chemical reaction takes place between gases and/or one or more gases and the substrate surface
v) desorption and diffusion away from substrate surface of reaction byproducts.

In the case of plasma enhanced CVD the gases are directed so as to diffuse through a plasma. Any appropriate plasma may be utilised. Non-thermal equilibrium plasma such as for example glow discharge plasma may be utilised. The glow discharge may be generated at low pressure, i.e. vacuum glow discharge or in the vicinity of atmospheric pressure - atmospheric pressure glow discharge, however in respect of the present invention the latter is preferred. Glow discharge plasma is generated in a gas, such as helium by a high frequency electric field.

Typically the plasma is generated in a gap between two electrodes, at least one of which is encased or coated or the like in a dielectric material. PE-CVD may be utilised at any suitable temperature e.g. a plasma a temperature of from room temperature to 500°C.

Yasuda, H. Plasma Polymerization; Academic Press: Orlando, 1985 describes how vacuum glow discharge has been used to polymerise gas phase polymer precursors into continuous films. As an example, the plasma enhanced surface treatment and deposition of fluorocarbons has been investigated for the preparation of oleophobic surfaces since the 1970's. Initially, simple fluorocarbon gas precursors such as carbon tetrafluoride were used; this improved hydrophobicity but did not significantly improve oleophobicity. Subsequently, as described in EP 0049884 higher molecular weight fluorinated precursors such as the perfluoroalkyl substituted acrylates were used.

These early processes typically resulted in fragmentation of the precursor and insertion of fluorine into the surface rather than formation of a polymerised fluorocarbon coating. The development of pulsed plasma polymerization (or modulated discharge) as described in Ryan, M., Hynes, A., Badyal, J., Chem. Mater. 1996, 8(1), 37-42 and Chen, X., Rajeshwar, K., Timmons, R., Chen, J., Chyan, O., Chem. Mater. 1996, 8(5), 1067-77 produced polymerised coatings in which the properties and/or functionalities of the monomer are substantially retained resulting in the production of a polymeric coating retaining many properties of the bulk polymer. Coulson S.R., Woodward I.S., Badyal J.P.S., Brewer S.A., Willis C., Langmuir, 16, 6287-6293, (2000) describe the production of highly oleophobic surfaces using long chain perfluoroacrylate or perfluoroalkene precursors.

Vacuum glow discharge processes have been investigated as routes to encapsulation and controlled release for example Colter, K. D.; Shen, M.; Bell, A. T. Biomaterials, Medical Devices, and Artificial Organs (1977), 5(1), 13-24 describes a method where fluoropolymer coatings are applied to reduce the diffusion of a steroid active through a poly(dimethylsiloxane) elastomer. Kitade, Tatsuya; Kitamura, Keisuke; Hozumi, Kei. Chemical & Pharmaceutical Bulletin (1987), 35(11), 4410-17 describes the application of vacuum glow discharge plasma to coat a powdered active with a PTFE based coating for controlled dissolution. WO 9910560 describes a further vacuum plasma method where precursor vapour is introduced to the plasma to produce coatings for the purpose of encapsulation.

Two significant drawbacks exist for vacuum plasma methods, firstly the necessity for a vacuum requires the coating process to be operated in a batch wise format, secondly the active must be introduced into the plasma as a vapour if the vacuum is to be maintained or the active is coated by conventional means and then in a separate step coated with an encapsulating plasma coating.

Both Atmospheric Pressure Glow Discharge (APGD) and Dielectric Barrier Discharge (DBD) offer an alternative homogeneous plasma source, which have many of the benefits of vacuum plasma methods, while operating at atmospheric pressure. The use of APGD was significantly developed 1980's, e.g. as described in Kanazawa S., Kogoma M., Moriwaki T., Okazaki S., J.Phys. D: Appl. Phys., 21, 838-840 (1988) and Roth J.R., Industrial Plasma Engineering Volume 2 Applications to Nonthermal Plasma Processing, Institute of Physics Publishing, 2001, pages 37-73. WO 01 59809 and WO 02 35576 describe a series of wide area APGD systems, which provide a uniform, homogeneous plasma at ambient pressure by application of a low frequency RF voltage across opposing parallel plate electrodes separated by ~10 mm. The ambient pressure and temperature ensures compatibility with open perimeter, continuous, on-line processing.

Considerable work has been done on the stabilisation of atmospheric pressure glow discharges, described in "Appearance of stable glow discharge in air, argon, oxygen and nitrogen at atmospheric pressure using a 50 Hz source" by Satiko Okazaki, Masuhiro Kogoma, Makoto Uehara and Yoshihisa Kimura, J. Phys. D: Appl. Phys. 26 (1993) 889-892. Further, there is described in US 5414324 (Roth et al) the generation of a steady-state glow discharge plasma at atmospheric pressure between a pair of insulated metal plate electrodes spaced up to 5 cm apart and radio frequency (R.F). energised with a root mean square (rms) potential of 1 to 5 kV at 1 to 100 kHz. This patent specification describes the use of electrically insulated metallic plate electrodes. This patent specification also describes a number of problems relating to the use of plate electrodes and the need to discourage electrical breakdown at the tips of electrodes.

These ambient temperature, atmospheric plasma systems have also been used to demonstrate the deposition of plasma coatings from vapour phase monomers - in effect atmospheric PE-CVD. For example EP 0431951 describes surface treatment with silane and disilane vapour and US 6146724 describes the deposition of a barrier coating from siloxane vapour precursors.

WO 02/28548 describes a process for enabling the introduction of a solid or liquid precursor into an atmospheric pressure plasma discharge and/or an ionised gas stream resulting therefrom in order to form a coating on a substrate. Where the substrate comprises metal, ceramic, plastic, woven or non-woven fibres, natural fibres, synthetic fibres, cellulosic material and powders. The invention describes how the chemical properties of the reactive coating precursor are substantially retained.

In accordance with the present invention there is provided a method for forming an active material containing coating on a substrate, which substrate is a wipe, cloth or sponge for household use, or a water soluble household cleaning unit dose product, which method comprises the steps of :-
introducing one or more gaseous or atomised liquid and/or solid coating-forming materials which undergo chemical bond forming reactions within a plasma environment and one or more active materials which substantially do not undergo chemical bond forming reactions within a plasma environment , into an atmospheric or low pressure plasma discharge and/or an excited gas stream resulting therefrom, and ii) exposing the substrate to the resulting mixture of atomised coating-forming and at least one active material which are deposited onto the substrate surface to form a coating.

By household use is meant household hard surface cleaners (including but not limited to glass, ceramic, wood and plastics cleaners), household surface cleaners with antimicrobial and or disinfecting and or antiseptic activity, insecticides or insect repellents for household use, air care products including malodour neutralisers, anti-allergenic agents and fragrancing delivered into household and automotive air spaces, polishes (including but not limited to those for polishing the floor furniture, shoe and metal), automatic dishwashing products including "in machine" wash and pre/post-treatment products and fabric care products for water softening in washing machines, carpet cleaners and stain removal pre-wash treatments.

The resulting coating which is prepared comprises a coating of the substrate comprising a coating made from the plasma activated coating derived from the coating forming material having particles/molecules of the active material trapped/encapsulated within the coating.

Preferably the plasma utilised is at substantially atmospheric pressure.

Any suitable active material may be utilised providing it substantially does not undergo chemical bond forming reactions within a plasma. Examples of suitable active materials include anti-microbials (for example, quaternary ammonium and silver based), , anti-oxidant, ,diagnostic materials, anti-bacterials, anti-fungals, cosmetics, cleansers, aloe, and vitamins, dyestuffs and pigments, for example photochromic dyestuffs and pigments and catalysts.

The chemical nature of the active material(s) used in the present invention is/are generally not critical. They can comprise any solid or liquid material which can be bound in the composition and where appropriate subsequently released at a desired rate.

Active materials which may be employed include, for example, antiseptics, anti-fungals, anti-bacterials, anti-microbials, biocides, proteolytic enzymes or peptides.

The active may comprise non-toxic cleansers for example in a nanoparticle form such as nanoparticles of para-chloro-meta-xylenol (PCMX). non-toxic cleanser. Some examples of biocides are Aluminum Phenolsulfonate, Ammonium Phenolsulfonate, Bakuchiol, Benzalkonium Bromide, Benzalkonium Cetyl Phosphate, Benzalkonium Chloride, Benzalkonium Saccharinate, Benzethonium Chloride, Potassium Phenoxide, Benzoxiquine, Benzoxonium Chloride, Bispyrithione, Boric Acid, Bromochlorophene, Camphor Benzalkonium Methosulfate, Captan, Cetalkonium Chloride, Cetearalkonium Bromide, Cetethyldimonium Bromide, Cetrimonium Bromide, Cetrimonium Chloride, Cetrimonium Methosulfate, Cetrimonium Saccharinate, Cetrimonium Tosylate, Cetylpyridinium Chloride, Chloramine T, Chlorhexidine, Chlorhexidine Diacetate, Chlorhexidine Digluconate, Chlorhexidine Dihydrochloride, p-Chloro-m-Cresol, Chlorophene, p-Chlorophenol, Chlorothymol, Chloroxylenol, Chlorphenesin, Ciclopirox Olamine, Climbazole, Cloflucarban, Clotrimazole, Coal Tar, Colloidal Sulfur, o-Cymen-5-ol, Dequalinium Acetate, Dequalinium Chloride, Dibromopropamidine Diisethionate, Dichlorobenzyl Alcohol, Dichlorophene, Dichlorophenyl Imidazoldioxolan, Dichloro-m-Xylenol, Diiodomethyltolylsulfone, Dimethylol Ethylene Thiourea, Diphenylmethyl Piperazinylbenzimidazole, Domiphen Bromide, 7-Ethylbicyclooxazolidine, Fluorosalan, Formaldehyde, Glutaral, Hexachlorophene, Hexamidine, Hexamidine Diisethionate, Hexamidine Diparaben, Hexamidine Paraben, Hexetidine, Hydrogen Peroxide, Hydroxymethyl Dioxoazabicyclooctane, Ichthammol, Isopropyl Cresol, Lapyrium Chloride, Lauralkonium Bromide, Lauralkonium Chloride, Laurtrimonium Bromide, Laurtrimonium Chloride, Laurtrimonium Trichlorophenoxide, Lauryl Isoquinolinium Bromide, Lauryl Isoquinolinium Saccharinate, Laurylpyridinium Chloride, Mercuric Oxide, Methenamine, Methenammonium Chloride, Methylbenzethonium Chloride, Myristalkonium Chloride, Myristalkonium Saccharinate, Myrtrimonium Bromide, Nonoxynol-9 Iodine, Nonoxynol-12 Iodine, Olealkonium Chloride, Oxyquinoline, Oxyquinoline Benzoate, Oxyquinoline Sulfate, PEG-2 Coco-Benzonium Chloride, PEG-10 Coco-Benzonium Chloride, PEG-6 Undecylenate, PEG-8 Undecylenate, Phenol, o-Phenylphenol, Phenyl Salicylate, Piroctone Olamine, Sulfosuccinylundecylenate, Potassium o-Phenylphenate, Potassium Salicylate, Potassium Troclosene, Propionic Acid, PVP-Iodine, Quaternium-8, Quaternium-14, Quaternium-24, Sodium Phenolsulfonate, Sodium Phenoxide, Sodium o-Phenylphenate, Sodium Shale Oil Sulfonate, Sodium Usnate, Thiabendazole, 2,2'-Thiobis(4-Chlorophenol), Thiram, Triacetin, Triclocarban, Triclosan, Trioctyldodecyl Borate, Undecylenamidopropylamine Oxide, Undecyleneth-6, Undecylenic Acid, Zinc Acetate, Zinc Aspartate, Zinc Borate, Zinc Chloride, Zinc Citrate, Zinc Cysteinate, Zinc Dibutyldithiocarbamate, Zinc Gluconate, Zinc Glutamate, Zinc Lactate, Zinc Phenolsulfonate, Zinc Pyrithione, Zinc Sulfate, and Zinc Undecylenate.

Some examples of oxidizing materials which may be utilized as the active material in a composition in accordance with the present invention include Ammonium Persulfate, Potassium Bromate, Potassium Caroate, Potassium Chlorate, Potassium Persulfate, Sodium Bromate, Sodium Chlorate, Sodium Iodate, Sodium Perborate, Sodium Persulfate and, Strontium Dioxide.

Some examples of reducing materials which may be utilized as the active material in a composition in accordance with the present invention include Ammonium Bisufite, Ammonium Sulfite, Ammonium Thioglycolate, Ammonium Thiolactate, Cystemaine HCl, Cystein, Cysteine HCl, Ethanolamine Thioglycolate, Glutathione, Glyceryl Thioglycolate, Glyceryl Thioproprionate, Hydroquinone, p-Hydroxyanisole, Isooctyl Thioglycolate, Magnesium Thioglycolate, Mercaptopropionic Acid, Potassium Metabisulfite, Potassium Sulfite, Potassium Thioglycolate, Sodium Bisulfite, Sodium Hydrosulfite, Sodium Hydroxymethane Sulfonate, Sodium Metabisulfite, Sodium Sulfite, Sodium Thioglycolate, Strontium Thioglycolate, Superoxide Dismutase, Thioglycerin, Thioglycolic Acid, Thiolactic Acid, Thiosalicylic Acid, and Zinc Formaldehyde Sulfoxylate.

Active flame retardants may also be included as the active material. These include for example halogen based flame-retardants such as decabromodiphenyloxide, octabromordiphenyl oxide, hexabromocyclododecane, decabromobiphenyl oxide, diphenyoxybenzene, ethylene bis- tetrabromophthalmide, pentabromoethyl benzene, pentabromobenzyl acrylate, tribromophenyl maleic imide, tetrabromobisphenyl A and derivatives thereof, bis-(tribromophenoxy) ethane, bis-(pentabromophenoxy) ethane, polydibomophenylene oxide, tribromophenylallyl ether, bis-dibromopropyl ether, tetrabromophthalic anhydride and derivatives, dibromoneopentyl gycol, dibromoethyl dibromocyclohexane, pentabromodiphenyl oxide, tribromostyrene, pentabromochlorocyclohexane, tetrabromoxylene, hexabromocyclododecane, brominated polystyrene, tetradecabromodiphenoxybenzene, trifluoropropene and PVC. Alternatively they may be phosphorous based flame-retardants such as (2,3-dibromopropyl)-phosphate, phosphorous, cyclic phosphates, triaryl phosphate, bis-melaminium pentate, pentaerythritol bicyclic phosphate, dimethyl methyl phosphate, phosphine oixide diol, triphenyl phosphate, tris- (2-chloroethyl) phosphate, phosphate esters such as tricreyl, trixylenyl, isodecyl diphenyl, ethylhexyl diphenyl, Phosphate salts of various amines such as ammonium phosphate, trioctyl, tributyl or tris-butoxyethyl phosphate ester. Other flame retardent actives may include tetraalkyl lead compounds such as tetraethyl lead, iron pentacarbonyl, manganese methyl cyclopentadienyl tricarbonyl, melamine and derivatives such as melamine salts, guanidine, dicayandiamide, silicones such as poldimethylsiloxanes, ammonium sulphamate, alumina trihydrate, magnesium hydroxide, or Alumina trihydrate

Some examples of UV light absorbing materials which may be utilized as the active material in a composition in accordance with the present invention include Acetaminosalol, Allatoin PABA, Benzalphthalide, Benzophenone, Benzophenone 1-12, 3-Benzylidene Camphor, Benzylidenecamphor Hydrolyzed Collagen Sulfonamide, Benzylidene Camphor Sulfonic Acid, Benzyl Salicylate, Bomelone, Bumetriozole, Butyl Methoxydibenzoylmethane, Butyl PABA, Ceria/Silica, Ceria/Silica Talc, Cinoxate, DEA-Methoxycinnamate, Dibenzoxazol Naphthalene, Di-t-Butyl Hydroxybenzylidene Camphor, Digalloyl Trioleate, Diisopropyl Methyl Cinnamate, Dimethyl PABA Ethyl Cetearyldimonium Tosylate, Dioctyl Butamido Triazone, Diphenyl Carbomethoxy Acetoxy Naphthopyran, Disodium Bisethylphenyl Tiamminotriazine Stilbenedisulfonate, Disodium Distyrylbiphenyl Triaminotriazine Stilbenedisulfonate, Disodium Distyrylbiphenyl Disulfonate, Drometrizole, Drometrizole Trisiloxane, Ethyl Dihydroxypropyl PABA, Ethyl Diisopropylcinnamate, Ethyl Methoxycinnamate, Ethyl PABA, Ethyl Urocanate, Etrocrylene Ferulic Acid, Glyceryl Octanoate Dimethoxycinnamate, Glyceryl PABA, Glycol Salicylate, Homosalate, Isoamyl p-Methoxycinnamate, Isopropylbenzyl Salicylate, Isopropyl Dibenzolylmethane, Isopropyl Methoxycinnamate, Menthyl Anthranilate, Menthyl Salicylate, 4-Methylbenzylidene, Camphor, Octocrylene, Octrizole, Octyl Dimethyl PABA, Octyl Methoxycinnamate, Octyl Salicylate, Octyl Triazone, PABA, PEG-25 PABA, Pentyl Dimethyl PABA, Phenylbenzimidazole Sulfonic Acid, Polyacrylamidomethyl Benzylidene Camphor, Potassium Methoxycinnamate, Potassium Phenylbenzimidazole Sulfonate, Red Petrolatum, Sodium Phenylbenzimidazole Sulfonate, Sodium Urocanate, TEA-Phenylbenzimidazole Sulfonate, TEA-Salicylate, Terephthalylidene Dicamphor Sulfonic Acid, Titanium Dioxide, TriPABA Panthenol, Urocanic Acid, and VA/Crotonates/Methacryloxybenzophenone-1 Copolymer.

Catalytically active materials which may be utilized as the active material in a composition in accordance with the present invention may include particles that contain metals such as Pt, Rh, Ag, Au, Pd, Cu, Ru, Ni, Mg, Co or other catalytically active metals. Mixtures of metals such as Pt-Rh, Rh-Ag, V-Ti or other well known mixtures may also be used. The metal may exist in it's elemental state, as a fine powder, or as a complex such as a metallocene, chloride, carbonyl, nitrate or other well known forms. Pure oxides such as CeOₓ, P₂O₅, TiO₂, ZrO₂, or mixed metal oxides such as aluminosilicates or perovskites can also give catalytic activity. Alternatively, non-metallic catalysts may be used. Examples of such non-metallic catalysts include sulphuric acid, acetic acid, sodium hydroxide or phosphoric acids. In the case of a catalyst or the like, the coating derived from the coating forming material may be a simple polymer designed to disperse and entrap active material and in the case where the active material is (e.g. a catalyst), or it may act to promote the activity of the catalyst material through well-known catalyst support interactions. Examples of such interactions are those found in Rh supported on ceria, Ni supported on alumina, Pt supported on Ce_{0.6}Zr_{0.4}O₂, Cr supported on titania or Pt-Pd supported on magnesium oxide.

Dispersing a conducting active material in a polymer matrix may give rise to conductive coatings to provide antistatic effects. The conductive material may comprise any conductive particle, typically of silver but alternative conductive particles might be used including gold, nickel, copper, assorted metal oxides and/or carbon including carbon nanotubes; or metallised glass or ceramic beads. Conductivity enhancing materials, such as those described in US 6,599,446 may also be added.

It is to be understood that the coating forming material in accordance with the present invention is a precursor material which is reactive within the atmospheric pressure plasma or as part of a PE-CVD process and can be used to make any appropriate coating, including, for example, a material which can be used to grow a film or to chemically modify an existing surface. The present invention may be used to form many different types of coatings. The type of coating which is formed on a substrate is determined by the coating-forming material(s) used, and the present method may be used to (co)polymerise coating-forming monomer material(s) onto a substrate surface.

The coating-forming material may be organic or inorganic, solid, liquid or gaseous, or mixtures thereof Suitable organic coating-forming materials include carboxylates, methacrylates, acrylates, styrenes, methacrylonitriles, alkenes and dienes, for example methyl methacrylate, ethyl methacrylate, propyl methacrylate, butyl methacrylate, and other alkyl methacrylates, and the corresponding acrylates, including organofunctional methacrylates and acrylates, including poly(ethyleneglycol) acrylates and methacrylates, glycidyl methacrylate, trimethoxysilyl propyl methacrylate, allyl methacrylate, hydroxyethyl methacrylate, hydroxypropyl methacrylate, dialkylaminoalkyl methacrylates, and fluoroalkyl (meth)acrylates, methacrylic acid, acrylic acid, fumaric acid and esters, itaconic acid (and esters), maleic anhydride, styrene, α-methylstyrene, halogenated alkenes, for example, vinyl halides, such as vinyl chlorides and vinyl fluorides, and fluorinated alkenes, for example perfluoroalkenes, acrylonitrile, methacrylonitrile, ethylene, propylene, allyl amine, vinylidene halides, butadienes, acrylamide, such as N-isopropylacrylamide, methacrylamide, epoxy compounds, for example glycidoxypropyltrimethoxysilane, glycidol, styrene oxide, butadiene monoxide, ethyleneglycol diglycidylether, glycidyl methacrylate, bisphenol A diglycidylether (and its oligomers), vinylcyclohexene oxide, conducting polymers such as pyrrole and thiophene and their derivatives, and phosphorus-containing compounds, for example dimethylallylphosphonate.

Suitable inorganic coating-forming materials include metals and metal oxides, including colloidal metals. Organometallic compounds may also be suitable coating-forming materials, including metal alkoxides such as titanates, tin alkoxides, zirconates and alkoxides of germanium and erbium. However, the present inventors have found that the present invention has particular utility in providing substrates with siloxane-based coatings using coating-forming compositions comprising silicon-containing materials. Suitable silicon-containing materials for use in the method of the present invention include silanes (for example, silane, alkylsilanes, alkylhalosilanes, alkoxysilanes) and linear (for example, polydimethylsiloxane) and cyclic siloxanes (for example, octamethylcyclotetrasiloxane), including organo-functional linear and cyclic siloxanes (for example, Si-H containing, halo-functional, and haloalkyl-functional linear and cyclic siloxanes, e.g. tetramethylcyclotetrasiloxane and tri(nonofluorobutyl)trimethylcyclotrisiloxane). A mixture of different silicon-containing materials may be used, for example to tailor the physical properties of the substrate coating for a specified need (e.g. thermal properties, optical properties, such as refractive index, and viscoelastic properties).

The substrate to be coated may comprise any material suitable for forming into a wipe, cloth or sponge, for example plastics for example thermoplastics such as polyolefins e.g. polyethylene, and polypropylene, polycarbonates, polyurethanes, polyvinylchloride, polyesters (for example polyalkylene terephthalates, particularly polyethylene terephthalate), polymethacrylates (for example polymethylmethacrylate and polymers of hydroxyethylmethacrylate), polyepoxides, polysulphones, polyphenylenes, polyetherketones, polyimides, polyamides, polystyrenes, polyfluoroalkanes such as PTFE, poly(siloxanes) such as poly(dimethylsiloxanes), phenolic, epoxy and melamine-formaldehyde resins, and blends and copolymers thereof. Preferred organic polymeric materials are polyolefins, in particular polyethylene and polypropylene.

The substrate is a wipe, cloth or sponge, or a water soluble household cleaning unit does product. The wipe, cloth or sponge may, for example, be for household cleaning, especially hard surface cleaning.

The wipe or cloth may be woven or non-woven, and may comprise synthetic or natural fibres or a mixture thereof, or be made of a sponge material. Typical materials for the fibres are cotton, cellulose, wool, polyethylene, polypropylene, acetate, polyamide, rayon, viscose and/or polyacrylonitrile. Reinforcing threads may be present, if desired. Typically the wipe has a weight of from 40 to 80g per m³, preferably 50 to 70g per m³, and a size of from 15 to 40 cm by 15 to 40 cm. The wipe, cloth or sponge may, if desired, be impregnated by a component such as water or a cleaning composition as disclosed in, for example, GB-A-2,368,590.
The sponge may, for example, be natural or synthetic.

The water soluble household cleaning unit dose product can be, for example, a water-soluble container comprising a fabric care, surface care or dishwashing composition such as a water-softening, or rinse aid, or a disinfectant, antibacterial or antiseptic composition or a refill composition for a trigger-type spray. The container may be made from a water-soluble film such as a polyvinyl alcohol (PVOH) film. The PVOH film may be partially or fully alcholized or hydrolysed, for example 40 to 100%, preferably 70 to 90%, more preferably 88 to 92% alcholized or hydrolysed polyvinyl acetate film. Examples of such unit dose products are given in WO 02/16222.

Any suitable means for generating the plasma may be utilised. Any conventional means for generating an atmospheric pressure plasma glow discharge may be used in the present invention, for example atmospheric pressure plasma jet, atmospheric pressure microwave glow discharge and atmospheric pressure glow discharge.

Preferably the current invention utilises equipment similar to that described in WO 02/28548, wherein liquid based polymer precursors are introduced as an aerosol into an atmospheric plasma discharge or the excited species therefrom. However, the reactive polymer precursors are also mixed with "active" materials, which are nonreactive within the atmospheric glow discharge. The "active" materials are chosen as they substantially avoid reactions in the plasma environment. One advantage of this method compared to WO 02/28548 is that "active" materials, which substantially do not undergo chemical bond forming reactions within a plasma environment, may be incorporated into the plasma deposited coating without degradation of the "active" properties. Thus an "active" coating can be readily prepared by atmospheric PE-CVD as well as when using liquid precursors.

An additional advantage of this method is that diffusion of the "active" from the coating may be controlled by the properties of the plasma coating. Diffusion is hindered by increased cross-linking, which may give rise to controlled release properties. Diffusion may also be hindered to the point where "active" is not released from the coating, either by increasing the cross-link density or over coating with a barrier coating. An advantage of the present invention over the prior art is that both liquid and solid atomised coating-forming materials may be used to form substrate coatings, due to the method of the present invention taking place under conditions of atmospheric pressure. Furthermore the coating-forming materials can be introduced into the plasma discharge or resulting stream in the absence of a carrier gas, i.e. they can be introduced directly by, for example, direct injection, whereby the coating forming materials are injected directly into the plasma.

For typical plasma generating apparatus, the plasma is generated between a pair of electrodes within a gap of from 3 to 50mm, for example 5 to 25mm. Thus, the present invention has particular utility for coating films, fibres and powders. The generation of steady-state glow discharge plasma at atmospheric pressure is preferably obtained between adjacent electrodes which may be spaced up to 5 cm apart, dependent on the process gas used. The electrodes being radio frequency energised with a root mean square (rms) potential of 1 to 100 kV, preferably between 1 and 30 kV at 1 to 100 kHz, preferably at 15 to 50 kHz. The voltage used to form the plasma will typically be between 1 and 30 kVolts, most preferably between 2.5 and 10 kV however the actual value will depend on the chemistry/gas choice and plasma region size between the electrodes.

Any suitable electrode systems may be utilised. Each electrode may comprise a metal plate or metal gauze or the like retained in a dielectric material or may, for example, be of the type described the applicants co-pending application WO 02/35576 wherein there are provided electrode units containing an electrode and an adjacent a dielectric plate and a cooling liquid distribution system for directing a cooling conductive liquid onto the exterior of the electrode to cover a planar face of the electrode. Each electrode unit comprises a watertight box having one side in the form of a dielectric plate to which a metal plate or gauze electrode is attached on the inside of the box. There is also a liquid inlet and a liquid outlet fitted to a liquid distribution system comprising a cooler and a recirculation pump and/or a sparge pipe incorporating spray nozzles. The cooling liquid covers the face of the electrode remote from the dielectric plate. The cooling conductive liquid is preferably water and may contain conductivity controlling compounds such as metal salts or soluble organic additives. Ideally, the electrode is a metal plate or mesh electrode in contact with the dielectric plate. The dielectric plate extends beyond the perimeter of the electrode and the cooling liquid is also directed across the dielectric plate to cover at least that portion of dielectric bordering the periphery of the electrode. Preferably, all the dielectric plate is covered with cooling liquid. The water acts to electrically passivate any boundaries, singularities or nonuniformity in the metal electrodes such as edges, corners or mesh ends where the wire mesh electrodes are used.

In another alternative system each electrode may be of the type described the applicants co-pending application No PCT/EP2004/001756 which was published after the priority date of the present application. In PCT/EP2004/001756 each electrode comprises a housing having an inner and outer wall, wherein at least the inner wall is formed from a dielectric material, and which housing contains an at least substantially non-metallic electrically conductive material in direct contact with the inner wall instead of the "traditional" metal plate or mesh. Electrodes of this type are preferred because the inventors have identified that by using electrodes in accordance with the present invention to generate a Glow Discharge, the resulting homogeneous glow discharge can be generated with reduced inhomogeneities when compared to systems utilizing metal plate electrodes. A metal plate is never fixed directly to the inner wall of an electrode in the present invention and preferably, the non-metallic electrically conductive material is in direct contact with the inner wall of the electrode.

Dielectric materials referred to in the present application may be of suitable type examples include but are not restricted to polycarbonate, polyethylene, glass, glass laminates, epoxy filled glass laminates and the like. Preferably, the dielectric has sufficient strength in order to prevent any bowing or disfigurement of the dielectric by the conductive material in the electrode. Preferably, the dielectric used is machinable and is provided at a thickness of up to 50mm in thickness, more preferably up to 40mm thickness and most preferably 15 to 30mm thickness. In instances where the selected dielectric is not sufficiently transparent, a glass or the like window may be utilized to enable diagnostic viewing of the generated plasma.

The electrodes may be spaced apart by means of a spacer or the like, which is preferably also made from a dielectric material which thereby effects an increase in the overall dielectric strength of the system by eliminating any potential for discharge between the edges of the conductive liquid.

The substantially non-metallic electrically conductive material may be a liquid such as a polar solvent for example water, alcohol and/or glycols or aqueous salt solutions and mixtures thereof, but is preferably an aqueous salt solution. When water is used alone, it preferably comprises tap water or mineral water. Preferably, the water contains up to a maximum of about 25% by weight of a water soluble salt such as an alkali metal salt, for example sodium or potassium chloride or alkaline earth metal salts. This is because the conductive material present in such an electrode has substantially perfect conformity and thereby a perfectly homogeneous surface potential at the dielectric surface.

Alternatively, the substantially non-metallic electrically conductive material may be in the form of one or more conductive polymer compositions, which may typically be supplied in the form of pastes. Such pastes are currently used in the electronics industry for the adhesion and thermal management of electronic components, such as microprocessor chip sets. These pastes typically have sufficient mobility to flow and conform to surface irregularities.

Suitable polymers for the conductive polymer compositions in accordance with the present invention may include silicones, polyoxypolyeolefin elastomers, a hot melt based on a wax such as a, silicone wax, resin/polymer blends, silicone polyamide copolymers or other silicone-organic copolymers or the like or epoxy, polyimide, acrylate, urethane or isocyanate based polymers. The polymers will typically contain conductive particles, typically of silver but alternative conductive particles might be used including gold, nickel, copper, assorted metal oxides and/or carbon including carbon nanotubes; or metallised glass or ceramic beads. Specific examples polymers which might be used include the conductive polymer described in EP 240648 or silver filled organopolysiloxane based compositions such as Dow Corning ^{®} DA 6523, Dow Corning ^{®} DA 6524, Dow Coming ^{®} DA 6526 BD, and Dow Corning ^{®} DA 6533 sold by Dow Corning Corporation or silver filled epoxy based polymers such as Ablebond^{®} 8175 from (Ablestik Electronic Materials & Adhesives) Epo-Tek^{®} H20E-PFC or Epo-Tek^{®} E30 (Epoxy Technology Inc).

One example of the type of assembly which might be used on an industrial scale with electrodes in accordance with the present invention is wherein there is provided an atmospheric pressure plasma assembly comprising a first and second pair of parallel spaced-apart electrodes in accordance with the present invention, the spacing between inner plates of each pair of electrodes forming a first and second plasma zone wherein the assembly further comprises a means of transporting a substrate successively through said first and second plasma zones and an atomiser adapted to introduce an atomised liquid or solid coating making material into one of said first or second plasma zones. The basic concept for such equipment is described in the applicant's co-pending application WO 03/086031.which is incorporated herein by reference.

In a preferred embodiment, the electrodes are vertically arrayed.

As has been previously described herein one major advantage of the use of liquids for conducting materials is that each pair of electrodes can have a different amount of liquid present in each electrode resulting in a different sized plasma zone and therefore, path length and as such potentially a different reaction time for a substrate when it passes between the different pairs of electrodes. This might mean that the period of reaction time for a cleaning process in the first plasma zone may be different from path length and /or reaction time in the second plasma zone when a coating is being applied onto the substrate and the only action involved in varying these is the introduction of differing amounts of conducting liquid into the differing pairs of electrodes. Preferably, the same amount of liquid is used in each electrode of an electrode pair where both electrodes are as hereinbefore described.

Whilst the atmospheric pressure glow discharge assembly may operate at any suitable temperature, it preferably operates at a temperature between room temperature (20 ° C) and 70° C and is typically utilized at a temperature in the region of 30 to 50 ° C.

The coating-forming material may be atomised using any conventional means, for example an ultrasonic nozzle. The material to be atomised is preferably in the form of a liquid, a solid or a liquid/solid slurry. The atomiser preferably produces a coating-forming material drop size of from 10 to 100µm, more preferably from 10 to 50µm. Suitable atomisers for use in the present invention are ultrasonic nozzles from Sono-Tek Corporation, Milton, New York, USA or Lechler GmbH of Metzingen Germany. The apparatus of the present invention may include a plurality of atomisers, which may be of particular utility, for example, where the apparatus is to be used to form a copolymer coating on a substrate from two different coating-forming materials, where the monomers are immiscible or are in different phases, e.g. the first is a solid and the second is gaseous or liquid.

Preferably where suitable the active material is introduced into the system using the same atomiser(s) with which the coating forming material is introduced. However, the active material may be introduced into the system via a second or second series of atomisers or other introducing means, preferably simultaneously with the introduction of the coating-forming material. Any suitable alternative introducing means may be utilised such as for example compressed gas and/or gravity feed powder feeders. Where a carrier gas is used any suitable carrier gas may be utilised although helium is preferred.

The process gas used to generate a plasma suitable for use in the present invention may be any suitable gas but is preferably an inert gas or inert gas based mixture such as, for example helium, a mixture of helium and argon and an argon based mixture additionally containing ketones and/or related compounds. These process gases may be utilized alone or in combination with potentially reactive gases such as, for example, nitrogen, ammonia, O₂, H₂O, NO₂, air or hydrogen. Most preferably, the process gas will be Helium alone or in combination with an oxidizing or reducing gas. The selection of gas depends upon the plasma processes to be undertaken. When an oxidizing or reducing process gas is required, it will preferably be utilized in a mixture comprising 90 - 99% noble gas and 1 to 10% oxidizing or reducing gas.

Under oxidising conditions the present method may be used to form an oxygen containing coating on the substrate. For example, silica-based coatings can be formed on the substrate surface from atomised silicon-containing coating-forming materials. Under reducing conditions, the present method may be used to form oxygen free coatings, for example, silicon carbide based coatings may be formed from atomised silicon containing coating forming materials.

In a nitrogen containing atmosphere nitrogen can bind to the substrate surface, and in an atmosphere containing both nitrogen and oxygen, nitrates can bind to and/or form on the substrate surface. Such gases may also be used to pre-treat the substrate surface prior to exposure to a coating forming substance. For example, oxygen containing plasma treatment of the substrate may provide improved adhesion with the applied coating. The oxygen containing plasma being generated by introducing oxygen containing materials to the plasma such as oxygen gas or water.

In one embodiment the coated substrate of the present invention may be coated with a plurality of layers of differing composition. These may be applied by passing the substrate relative to a plurality of plasma regions or by repeatedly passing the substrate or partially coated substrate repeatedly relative to the plasma regions. Where appropriate the substrate or the plasma system may move relative to the other. Any suitable number of cycles or plasma zones may be utilised in order to achieve the appropriate multi-coated substrates. The substrate may pass through a plasma zone, adjacent a plasma zone through or remote from the excited gas stream or even remote thereof such that the substrate may be maintained outside the region effected by the plasma and/or excited gas stream.

For example, the substrate utilised in accordance to the present invention may be subjected to a plurality of plasma regions, each of which can function differently e.g. a first plasma region might be utilised as a means of oxidising the substrate surface (in for example, an oxygen/Helium process gas) or as a means of applying a first coating and the application of an active material containing coating may take place in a second plasma region which may or may not be post-treated with for example the addition of a further protective coating. The method of the present invention is therefore suitable to any number of required coating layers as required for the end use concerned.

In a still further embodiment where a substrate is to be coated, rather than having a multiple series of plasma assemblies, a single plasma assembly may be utilised with a means for varying the materials passing through the plasma zone formed between the electrodes. For example, initially the only substance passing through the plasma zone might be the process gas such as helium which is excited by the application of the potential between the electrodes to form a plasma zone. The resulting helium plasma may be utilised to clean and/or activate the substrate which is passed through or relative to the plasma zone. Then one or more coating forming precursor material(s) and the active material may be introduced and the one or more coating forming precursor material(s) are excited by passing through the plasma zone and treating the substrate. The substrate may be moved through or relative to the plasma zone on a plurality of occasions to effect a multiple layering and where appropriate the composition of the coating forming precursor material(s) may be varied by replacing, adding or stopping the introduction of one or more for example introducing one or more coating forming precursor material(s) and/or active materials.

Any suitable non-thermal equilibrium plasma equipment may be used to undertake the method of the present invention, however atmospheric pressure glow discharge, dielectric barrier discharge (DBD), low pressure glow discharge, which may be operated in either continuous mode or pulse mode are preferred.

The plasma equipment may also be in the form of a plasma jet as described in WO 03/085693. Where the substrate is placed downstream and remote from the plasma source.

Any conventional means for generating an atmospheric pressure glow discharge may be used in the method of the present invention, for example atmospheric pressure plasma jet, atmospheric pressure microwave glow discharge and atmospheric pressure glow discharge. Typically, such means will employ helium as the process gas and a high frequency (e.g.> 1kHz) power supply to generate a homogeneous glow discharge at atmospheric pressure via a Penning ionisation mechanism, (see for example, Kanazawa et al, J.Phys. D: Appl. Phys. 1988, 21, 838, Okazaki et al, Proc. Jpn. Symp. Plasma Chem. 1989, 2, 95, Kanazawa et al, Nuclear Instruments and Methods in Physical Research 1989, B37/38, 842, and Yokoyama et al., J. Phys. D: Appl. Phys. 1990, 23, 374).

In the case of low pressure glow discharge plasma, liquid precursor and the active material is preferably either retained in a container or is introduced into the reactor in the form of an atomised liquid spray as described above. The low pressure plasma may be performed with liquid precursor and/or active material heating and/or pulsing of the plasma discharge, but is preferably carried out without the need for additional heating. If heating is required, the method in accordance with the present invention using low pressure plasma techniques may be cyclic, i.e. the liquid precursor is plasma treated with no heating, followed by heating with no plasma treatment, etc., or may be simultaneous, i.e. liquid precursor heating and plasma treatment occurring together. The plasma may be generated by way of the electromagnetic radiations from any suitable source, such as radio frequency, microwave or direct current (DC). A radio frequency (RF) range between 8 and 16 MHz is suitable with an RF of 13.56 MHz preferred. In the case of low pressure glow discharge any suitable reaction chamber may be utilized. The power of the electrode system may be between 1 and 100 W, but preferably is in the region of from 5 to 50 W for continuous low pressure plasma techniques. The chamber pressure may be reduced to any suitable pressure for example from 0.1 to 0.001mbar but preferably is between 0.05 and 0.01 mbar.

A particularly preferred pulsed plasma treatment process involves pulsing the plasma discharge at room temperature. The plasma discharge is pulsed to have a particular "on" time and "off" time, such that a very low average power is applied, for example a power of less than 10W and preferably less than 1W. The on-time is typically from 10 to 10000µs, preferably 10 to 1000µs, and the off-time typically from 1000 to 10000µs, preferably from 1000 to 5000µs. Atomised liquid precursors and the active material(s) may be introduced into the vacuum with no additional gases, i.e. by direct injection, however additional process gases such as helium or argon may also be utilized as carriers where deemed necessary.

In the case of the low pressure plasma options the process gas for forming the plasma may be as described for the atmospheric pressure system but may alternatively not comprise noble gases such as helium and/or argon and may therefore purely be oxygen, air or an alternative oxidising gas.

The present invention will now be illustrated in detail with reference to the accompanying figures drawing and the Examples, in which :-
Figure 1 is a general view of a plasma generating unit as used in the Examples hereinbelow
Figure 2 is a High resolution carbon (C I s) spectra for cetalkonium chloride deposited in a) acrylic acid, b) PEG methacrylate. The x-axis is binding energy (eV) [(a) starts at 294 and ends at 282, (b) starts at 29² and ends at 282]. The y-axis is CPS, starting at 0 and ending at 20 x 10³ (a) and 25 x 10³ (b).

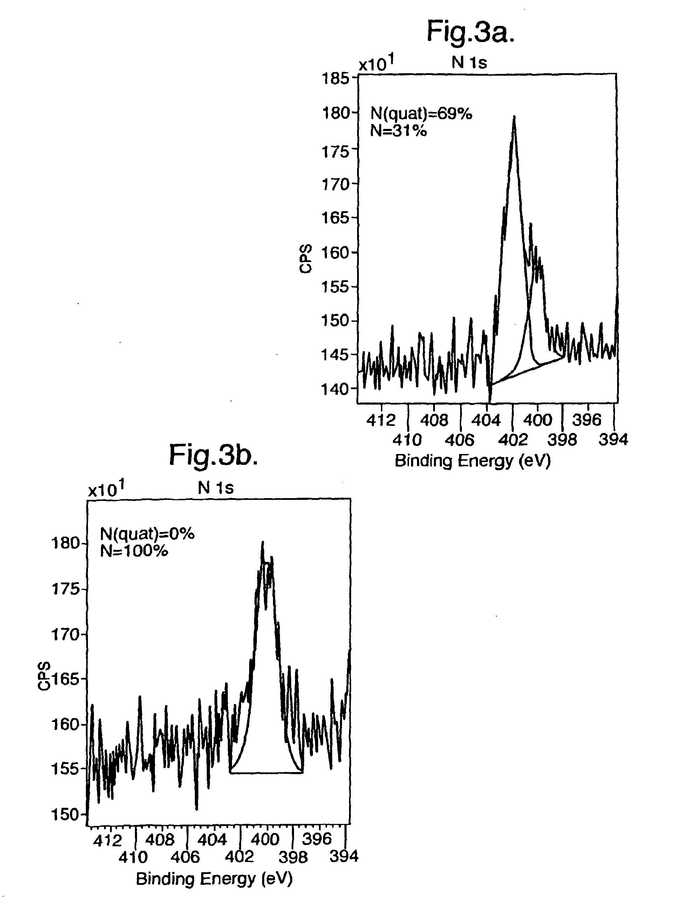

Figure 3 is a High resolution nitrogen (N Is) spectrum for Cetylalkonium chloride deposited in acrylic acid a) before washing, b) after washing in NaOH. The x-axis is binding energy (eV) (starts at 412 and ends at 394). The y-axis is CPS, starting at 0 and ending at 195 x 10³.

### EXPERIMENTAL

### Sample Preparation

Solid salts of cetalkonium chloride, benzalkonium chloride and cetyl pyridinium chloride (actives) were dissolved in acrylic acid or polyethylene glycol (PEG) methacrylates (coating-forming materials as described in Table 1

**Table 1: Composition of quaternary salt solutions**

| Solid | Weight (g) | Solvent | Weight (g) |
|---|---|---|---|
| Cetalkonium chloride | 0.38 | Acrylic acid | 16.1 |
| Benzalkonium chloride | 0.40 | Acrylic acid | 16.0 |
| Cetylpyridinium chloride | 0.29 | Acrylic acid | 12.0 |
| | | PEG methacrylate | 16.0 |
| Cetalkonium chloride | 0.48 | | |
| | | PEG dimethacrylate | 16.3 |
| Benzalkonium chloride | 0.25 | PEG methacrylate | 9.6 |
| | | PEG dimethacrylate | 6.5 |
| | | PEG methacrylate | 8.0 |
| Cetylpyridinium chloride | 0.25 | PEG dimethacrylate | 7.2 |
| | | Acrylic acid | 4.5 |

The chemical structures for the salts are given below

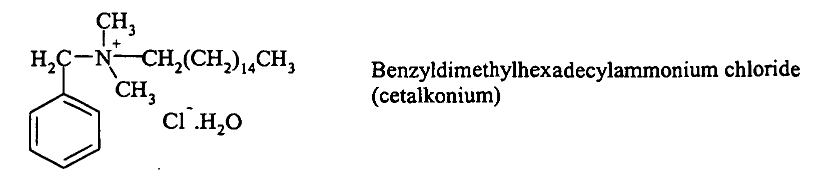

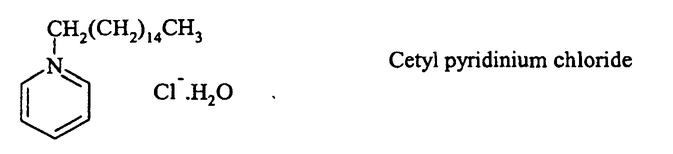

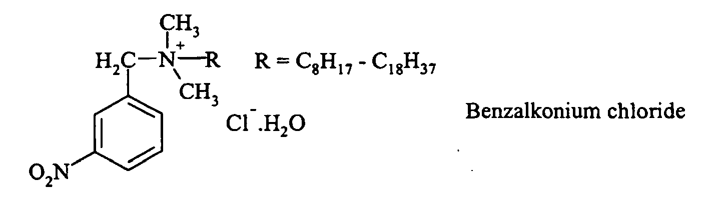

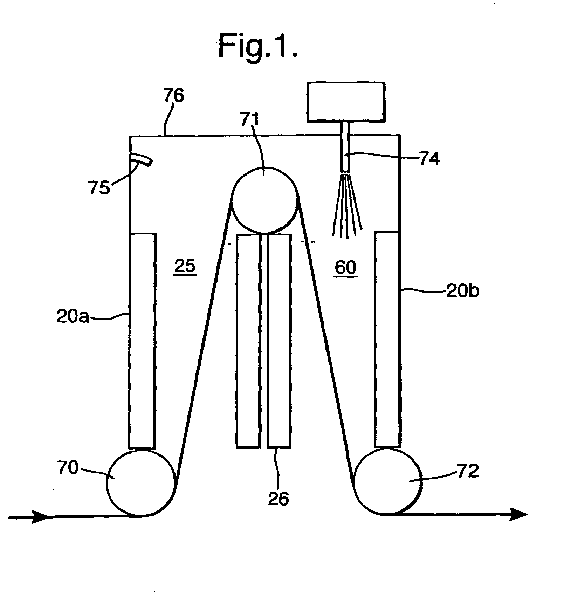

The precursor solutions comprising the coating-forming material and the active were then deposited onto polypropylene and polyester fabric substrates using an atmospheric pressure glow discharge assembly of the type shown in Fig.1.

Referring now to Fig. 1, the flexible polypropylene and polyester fabric substrate was transported through the plasma assembly by means of guide rollers 70, 71 and 72. A helium process gas inlet 75, an assembly lid 76 and an ultrasonic nozzle 74 for introducing atomised precursor solutions into plasma region 60 are provided. Plasma power used in both plasma regions varied between 0.4 and 1.0 kW.

In use a 100mm wide web of flexible substrate was transported through the plasma assembly at a speed of speed was varied between 1 and 4 mmin⁻¹. The substrate was initially directed to and over guide roller 70 through plasma region 25 between electrodes 20a and 26. The plasma generated between electrodes 20a and 26 in plasma region 25 was utilised as a cleaning helium plasma, i.e. no reactive material is directed into plasma region 25. Helium was introduced into the system by way of inlet 75. Lid 76 is placed over the top of the system to prevent the escape of helium as it is lighter than air. Upon leaving plasma region 25 the plasma cleaned substrate passes over guide 71 and is directed down through plasma region 60, between electrodes 26 and 20b and over roller 72. Plasma region 60 however is utilised to coat the substrate with plasma treated precursor solution introduced in a liquid form through ultrasonic nozzle introduced at a rate of between 25 - 50 µLmin⁻¹.

The precursor solution is itself plasma treated when passing through plasma region 60 generating a coating for the substrate in which the actives are retained. The coated substrate then passes through plasma region 60 and is coated and then is transported over roller 72 and is collected or further treated with additional plasma treatments. Rollers 70 and 72 may be reels as opposed to rollers. Having passed through is adapted to guide the substrate into plasma region25 and on to roller 71.

Table 2 describes the coating conditions used to prepare the samples, along with the corresponding analytical reference.

**Table 2: Coating conditions**

| Coating Conditions | Example Reference |
|---|---|
| Cetalkonium chloride/Acrylic acid 0.4 kW, 25 µlmin⁻¹ | 1a |
| Cetalkonium chloride/Acrylic acid 1.0 kW, 25 µlmin⁻¹ | 1b |
| Cetalkonium chloride/Acrylic acid 0.4 kW, 50 µlmin⁻¹ | 1c |
| Cetalkonium chloride/Acrylic acid 0.4 kW, 50 µlmin⁻¹ | 1d |
| Cetyl pyridinium chloride/Acrylic acid 1.0 kW, 25 µlmin⁻¹ | 1e |
| Cetyl pyridinium chloride/Acrylic acid 0.4 kW, 25 µlmin⁻¹ | 1f |
| Benzalkonium chloride/Acrylic acid 1.0 kW, 25 µlmin⁻¹ | 1g |
| Benzalkonium chloride/Acrylic acid 0.4 kW, 25 µlmin⁻¹ | 1h |
| Cetalkonium chloride/PEG acrylate 1.0 kW, 25 µlmin⁻¹ | 1i |
| Cetalkonium chloride/Acrylic acid 0.4 kW, 25 µlmin⁻¹ | 1j |

Samples were then washed by immersing a piece of coated film in the one of the
following solutions for 10 minutes at ambient temperature:

| | |
|---|---|
| pH 2 | 0.01M HCl |
| pH 7 | HPLC grade water |
| pH 12 | 0.01M NaOH |

All samples were then submitted for X-ray Photoelectron Spectroscopy (XPS) analysis which involves the irradiation of a sample with soft X-rays, and the energy analysis of photoemitted electrons that are generated close to the sample surface. XPS has the ability to detect all elements (with the exception of hydrogen and helium) in a quantitative manner from an analysis depth of less than 10nm. In addition to elemental information, XPS is also used to probe the chemical state of elements through the concept of binding energy shift. All values quoted in this report are an average of at least three different analyses.

| | |
|---|---|
| Instrument: | Kratos Analytical Axis Ultra |
| Sampling: | Monochromated A1 K X-rays |
| Spectra Acquired: | Survey, Na Is, O Is, N Is, C 1s |

### Anti-microbial testing

Anti-microbial testing was carried out using a modified version of ISO846 norm ("Plastics - Evaluation of the action of microorganisms"). Fabric and plastic samples were exposed to a mixed suspension of fungal spores in the presence of a complete medium, for a specified period of time (4 weeks) and in specified conditions of temperature (28°C ± 1°C) and humidity. The dishes were examined every 2 days in order to ensure spore viability. The final and official examination is performed after 4 incubation weeks. The broad spectrum efficiency of a material is determined by the "growth rating" scale from 0 to 5, in Table 3. This scale measured the extent to which visible fungal growth is inhibited on the material sample being tested.

**Table 3: Evaluation criteria for microbial tests**

| Intensity of growth | Evaluation |
|---|---|
| 0 | No growth apparent under the stereomicroscope. |
| 1 | No growth visible to the naked eye, but clearly visible under the stereomicroscope. |
| 2 | Growth visible to the naked eye, covering up to 25% of the test surface. |
| 3 | Growth visible to the naked eye, covering up to 50% of the test surface. |
| 4 | Considerable growth, covering more than 50% of the test surface. |
| 5 | Heavy growth, covering the entire test surface (=zero protection). |

The examples above demonstrate the incorporation of a quaternary ammonium surfactant (anti-microbial) into a polyethylene glycol PEG coating what substrate. The coating is resistant to water, acid and base washing.

All samples coated with the quaternary salt solutions gave rise to clear, hydrophilic coatings with good substrate coverage. XPS analysis was used to probe the surface chemistry of the deposited coatings. The plasma deposition process was shown to produce polymerised coatings on the substrate surface with good retention of the precursor functionality.

### Coated Samples

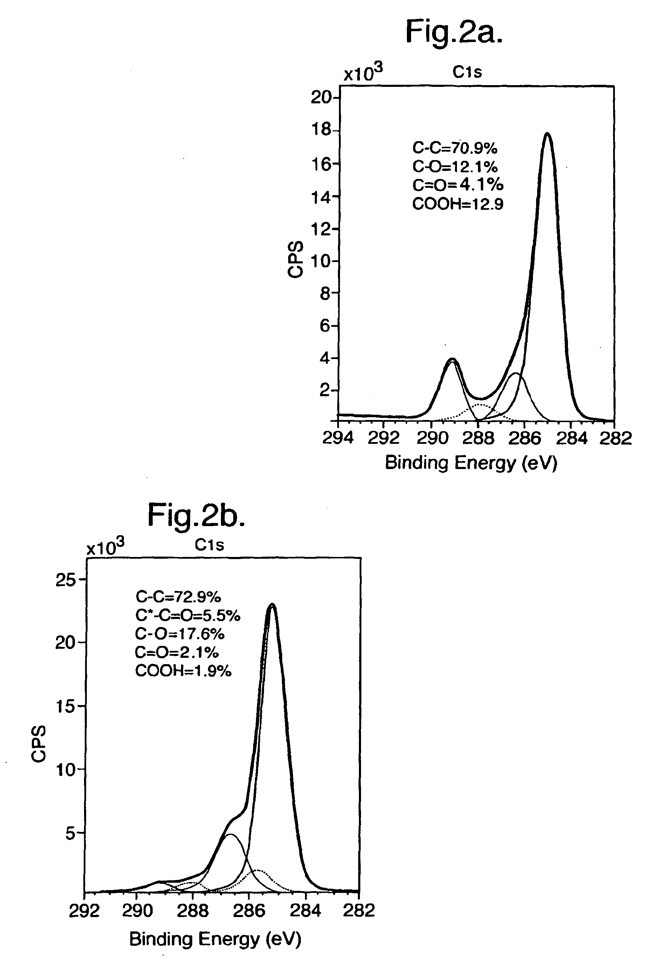

Fig. 2a shows a representative carbon (C Is) spectrum for polymerised acrylic acid based precursors. The C 1s spectrum shows both C-C chains and retention of COOH functionality. Some oxidation of the precursor was also observed, resulting in the presence of small quantities of C-O and C=O species. Investigation of the high resolution C1s spectra revealed very similar chemistry to that previously reported for acrylic acid derived plasma coatings. Compositional analysis for each sample is included in Table 4. Fig. 2b shows a C1s spectrum for a PEG acrylate based coating, displaying good retention of glycol functionality. The carbon chemistry for these samples may be found in Table 6.

In addition to the polymerised solvent, all samples contained 1 - 2% nitrogen, arising from the quaternary ammonium salt. High-resolution spectra revealed that the quaternary ammonium structure was retained during the plasma deposition process. Fig.2a shows a typical spectrum for polymerised salts in acrylic acid. The nitrogen (N 1s) core level shows a peak in the region of 398 - 404 eV. Fitting synthetic peaks to the core level required two overlapping peaks. The main peak at ~ 402eV is attributed to nitrogen in a quaternary ammonium structure. The second peak at - 400eV is attributed to a neutral NR₃ chemistry. The relative concentration of the quaternary ammonium salts was found to vary between 45 and 73% of the total N content, as is evident from Table 5 and 7.

**Table 4: Chemical environment of carbon for quaternary ammonium salts in acrylic acid**

| | C-C | C-O | C=O | COOH |
|---|---|---|---|---|
| 1a | 69.6 | 10.7 | 3.1 | 16.7 |
| 1b | 70.9 | 12.1 | 4.1 | 12.9 |
| 1c | 69.9 | 9.6 | 3.5 | 17.1 |
| 1d | 72.1 | 6.4 | 2.6 | 18.9 |
| 1e | 70.9 | 10.2 | 3.5 | 15.5 |
| 1f | 72.0 | 7.3 | 2.6 | 18.2 |
| 1g | 73.3 | 10.5 | 3.5 | 12.8 |
| 1h | 72.1 | 6.8 | 2.6 | 18.6 |

**Table 5: Chemical environment of nitrogen for quaternary ammonium salts in acrylic acid**

| | N (quat) | N |
|---|---|---|
| 1a | 67.0 | 33.0 |
| 1b | 69.0 | 31.0 |
| 1c | 62.5 | 37.5 |
| 1d | 55.7 | 44.3 |
| 1e | 59.7 | 40.3 |
| 1f | 53.1 | 46.9 |
| 1g | 59.5 | 40.5 |
| 1h | 72.9 | 27.1 |

**Table 6: Chemical environment of carbon for quaternary ammonium salts in PEG acrylate**

| | C-C | C*-CO | C-O | C=O | COOC |
|---|---|---|---|---|---|
| 1i | 64.7 | 6.1 | 24.0 | 2.9 | 2.4 |
| 1j | 72.9 | 5.5 | 17.6 | 2.1 | 1.9 |

**Table 7: Chemical environment of nitrogen for quaternary ammonium salts in PEG acrylate**

| | N (quat) | N |
|---|---|---|
| 1i | 48.5 | 51.6 |
| 1j | 44.7 | 55.3 |

### Wash Tests

Following deposition, samples were cut from the coated films and subjected to a variety of wash tests. Samples were washed in NaOH_{(aq)} -pH 12, Water -pH 7 and HCl_{(aq)} -pH 2.

In all cases, no nitrogen was lost during the washing process; all samples had between 1% and 2% nitrogen at the surface before and after washing. However, the relative concentration of quaternary ammonium salt did change as a function of the washing process. Table 8 contains representative data for a range of samples under different washing conditions.

Washing with either water or acid typically reduces the amount of N present as a quaternary ammonium (-NR₃⁺), the only exception being acid washing of cetyl pyridium chloride in acrylic acid. This indicates removal of free surfactant from the surface.

The sodium hydroxide wash was much more interesting, we have attributed this to deprotonation of the quaternary ammonium salt. In the case of cetalkonium chloride in acrylic acid, the -NR₃ is entirely deprotonated to the -NR₂ when washed in sodium hydroxide (Figure 2), indicating that the trapped surfactant is fully accessible to the applied wash solution. Deprotonation appear to be partially reversed when washed in acid. A similar effect is observed for cetalkonium chloride in PEG, except that deprotonation is fully reversed on washing in acid.

Cetyl pyridinium chloride in acrylic acid coatings are very stable to water washing, indicating good entrapment of the surfactant. On washing the coating with alkali, the NR₃⁺ is partially deprotonated, indicating that only ca. 40% of the NR₃⁺ is susceptible to alkali attack at the surface. This may be due to either the physical properties of the coating or the dissociation constants of the ammonium cation. The - NR₃⁺ reverts completely to -NR₂ on acid washing. A similar effect is observed for benzalkonium chloride in acrylic acid where it is partially converted to NR₂ on alkali wash, with nearly full reversion to -NR₃⁺ on acid wash.

Washing also changed the carbon chemistry of the coatings. The acrylic acid based coatings were severely altered by the washing procedures employed. Again, the sodium hydroxide wash proved to be the most aggressive, with the COOH functionality completely disappearing in some samples. Data for the sodium hydroxide washed samples are included in Tables 9-11. Although not as severe, all washing procedures lead to a reduction in the COOH peak.

The PEG based coatings were less susceptible to damage from the washing treatments. The sodium hydroxide altered the chemistry of the nitrogen component, but had limited effect on the PEG polymer. Water washing also had little effect. However, the HCl wash did have a dramatic effect on the C-O functionality, with most of the C-O species disappearing, as is evident from Table 12.

**Table 8: Nitrogen as quaternary ammonium with varying wash conditions**

| | % N as Quaternary Ammonium | | | | |
|---|---|---|---|---|---|
| | Coated | H₂O wash | HCl wash | NaOH wash | NaOH then HCl wash |
| Cetalkonium chloride in acrylic acid 0.4 kW, 25 µlmin⁻¹ | 67.0 | 49.2 | 58.3 | 0 | 34.1 |
| Cetyl Pyridinium chloride in acrylic acid 0.4 kW, 25 µlmin⁻¹ | 53.1 | 52.6 | 57.2 | 20.1 | 51.9 |
| Benzalkonium chloride in acrylic acid 0.4 kW, 25 µlmin⁻¹ | 72.9 | 40.0 | 54.7 | 38.9 | 61.4 |
| Cetalkonium chloride in PEG Methacrylate 0.4 kW, 25 µlmin⁻¹ | 44.7 | 40.1 | 48.4 | 0 | 46.6 |

**Table 9: Chemical environment of carbon for cetalkonium chloride deposited in acrylic acid using various washing conditions**

| Cetalkonium chloride in acrylic acid 0.4 kW, 25 µlmin⁻¹ | C-C | C*-C=O | C-O | C=O | C(O)OC | C(O)OH |
|---|---|---|---|---|---|---|
| coated | 72.1 | 0 | 6.4 | 2.6 | 0 | 18.9 |
| H₂O was | 72.0 | 11.1 | 5.7 | 2.8 | 4.0 | 4.5 |
| NaOH wash | 84.3 | 6.0 | 3.5 | 3.5 | 2.3 | 3.9 |
| HCl wash | 68.9 | 12.3 | 6.9 | 2.6 | 1.6 | 7.7 |
| NaOH then HCl wash | 84.5 | 4.3 | 6.4 | 1.5 | 1.0 | 2.3 |

**Table 10: Chemical environment of carbon for cetyl pyridinium chloride deposited in acrylic acid using various washing conditions**

| Cetyl pyridinum chloride in acrylic acid 0.4 kW, 25 µlmin⁻¹ | C-C | C*-C=O | C-O | C=O | C(O)OC | C(O)OH |
|---|---|---|---|---|---|---|
| coated | 72.0 | 0 | 7.3 | 2.6 | 0 | 18.2 |
| H₂O was | 77.8 | 8.6 | 5.2 | 1.9 | 2.6 | 3.9 |
| NaOH wash | 85.2 | 5.8 | 3.6 | 1.8 | 2.7 | 0.9 |
| HCl wash | 70.0 | 12.2 | 5.8 | 2.4 | 3.4 | 6.4 |
| NaOH then HCl wash | 86.9 | 4.6 | 4.2 | 0.8 | 1.1 | 2.5 |

**Table 11: Chemical environment of carbon for benzalkonium chloride deposited in acrylic acid using various washing conditions**

| Benzalkonium chloride in acrylic acid 0.4 kW, 25 µlmin⁻¹ | C-C | C*-C=O | C-O | C=O | C(O)OC | C(O)OH |
|---|---|---|---|---|---|---|
| coated | 72.1 | 0 | 6.8 | 2.6 | 0 | 18.6 |
| H₂O wash | 77.1 | 8.6 | 5.6 | 2.0 | 3.4 | 3.3 |
| NaOH wash | 89.2 | 3.6 | 3.5 | 2.4 | 1.4 | 0 |
| HCl wash | 72.3 | 11.3 | 5.0 | 2.3 | 2.9 | 6.3 |
| NaOH then HCl wash | 72.6 | 10.0 | 7.4 | 1.3 | 2.0 | 6.8 |

**Table 12: Chemical environment of carbon for cetalkonium chloride deposited in PEG acrylate using various washing conditions**

| Cetalkonium chloride in PEG methacrylate 0.4 kW, 25 µlmin⁻¹ | C-C | C*-C=O | C-O | C=O | C(O)OC |
|---|---|---|---|---|---|
| coated | 72.9 | 5.5 | 17.6 | 2.1 | 1.9 |
| H₂O wash | 75.3 | 3.6 | 17.9 | 1.6 | 1.6 |
| NaOH wash | 76.7 | 2.5 | 17.2 | 1.4 | 2.1 |
| HCl wash | 83.4 | 3.2 | 1.1 | 1.6 | 1.7 |
| NaOH then HCl wash | 80.3 | 2.6 | 14.6 | 0.9 | 1.7 |

### Anti-fungal activity of treated polyester fabrics

After 2 weeks of incubation, treated and untreated fabric specimens were entirely covered by microorganisms (growth rating = 5) - as shown in Figure 1. In general, fabric surface is a good support for microorganism adherence (i.e. the first step of a contamination process).

After 4 incubation weeks, moulds aggregated at the surface of fabric specimens in order to form a cell "skin". Using a scalpel, this cell skin was removed and the surface of fabric was analysed by stereomicroscopy. No trace of spores and mycelium was detected between stitches of treated and untreated fabric. All fabric samples presented a clean surface after removing the mould skin, because polyester is not an appropriate nutrient source for microorganisms.

After scraping the sample surface for removing moulds, all samples were soaked in alcohol and allowed to air dry before proceeding to a second visual observation. Results clearly showed that 4 samples presented a color change (pink color) and Table 13. Both untreated samples as well as samples treated with cetalkonium showed a color change after 4 weeks of microorganism attack, indicating degradation of the substrate had occurred. On the other hand, fabric samples treated with cetyl pyridinium and benzalkonium are very resistant to the treatment with microorganisms. No change of fabric texture and flexibility was observed.

**Table 13 Results of microbial testing**

| Sample | Colour change after treatment |
|---|---|
| Blank polyester fabric | Pink color |
| Acrylic acid on polyester fabric | Pink color |
| Cetalkonium chloride+ acrylic acid on polyester fabric | Pink color |
| Cetalkonium chloride + PEG methacrylate on polyester fabric | Pink color |
| Cetyl pyridinium chloride + acrylic acid on polyester fabric | No change |
| Cetyl pyridinium chloride + PEG methacrylate on polyester fabric | No change |
| Benzalkonium chloride + acrylic acid on polyester fabric | No change |
| Benzalkonium chloride + PEG methacrylate on polyester fabric | No change |

## Claims

1. A method for forming an active material containing coating on a substrate, which substrate is a wipe, cloth or sponge for household use, or a water soluble households cleaning unit dose product, which method comprises the steps of :-
i) introducing one or more gaseous or atomised liquid and/or solid coating-forming materials which undergo chemical bond forming reactions within a plasma environment and one or more active materials which substantially do not undergo chemical bond forming reactions within a plasma environment , into an atmospheric or low pressure plasma discharge and/or an excited gas stream resulting therefrom, and ii) exposing the substrate to the resulting mixture of atomised coating-forming and at least one active material which are deposited onto the substrate surface to form a coating.

2. A method in accordance with claim 1 wherein the coating forming material is introduced into the plasma discharge by means of one or more atomisers.

3. A method in accordance with claim 2 **characterised in that** each atomiser is an ultrasonic nozzle.

4. A method in accordance with any preceding claim wherein the active material is introduced into the plasma discharge through the same atomiser as the coating forming material.

5. A method in accordance with any one of claims 1 to 3 wherein the active material is introduced into the plasma discharge by way of a separate active introducing means.

6. A method in accordance with claim 5 wherein the active material introducing means is an atomiser or in the case of powders is a compressed gas or gravity powder feeder.

7. A method in accordance with any preceding claim wherein the substrate is passed through the plasma and/or the excited gas stream resulting therefrom.

8. A method in accordance with any preceding claim wherein the treatment of the substrate surface (1) is undertaken away from the plasma discharge plasma and/or the excited gas stream resulting therefrom.

9. A method in accordance with any preceding claim wherein the active material comprises one or more anti-microbials, enzymes, proteins, aloe, and vitamins, fragrances and/or catalyst.

10. A method in accordance with any preceding claim wherein the active material is a pharmaceutical material, or a cosme-ceuticalically active, therapeutically active or diagnostically active material.

11. A method in accordance with any preceding claim wherein the active material is an antiseptic, anti-fungal, anti-bacterial, anti-microbial, biocide, proteolytic enzyme or peptide.

12. A method in accordance with any one of claims 1 to 10 wherein the active material is a UV screening material, an anti-oxidant, a flame retardant, an anti-bacterial, an anti-fungal, a cleanser, aloe, a vitamin, a fragrance or a catalyst.

13. A method in accordance with any one of claims 1 to 10 wherein the active material is an absorbent, anti-oxidant, anti-static material, binder, buffering material, bulking material, chelating material, colourant, deodorant material, emollient, external analgesic, film former" fragrance ingredient, humectant, moisturizing material, opacifying material, oxidizing or reducing material, penetration enhancer, plasticizer, preservative, skin conditioning material, slip modifier, solubilizing material, solvent, surface modifier, surfactant or emulsifying material, suspending material, thickening material, viscosity controlling material including an increasing or decreasing material or a UV light absorber.

14. A method in accordance with any one of claims 1 to 10 wherein the active is a pesticidally, and/or fungicidally active material.

15. A method in accordance with any preceding claim wherein the substrate is plasma pretreated and/or post-treated.

16. A method in accordance with claim 15 wherein plasma post-treatment comprises the application of an additional active-free coating as a top coat.

17. A method in accordance with any preceding claim wherein a plurality of coatings containing one or more active materials is applied onto the substrate.

18. A method in accordance with any preceding claim wherein the coating is applied by means of a plasma enhanced chemical vapour deposition.

19. A method in accordance with any preceding claim wherein the substrate is a textile material for hard surface cleaning.

20. A method in accordance with any one of claims 1 to 18 wherein the substrate is a sponge for hard surface cleaning.

21. A method in accordance with any one of claims 1 to 18 wherein the substrate is a water soluble household cleaning unit dose product comprising a polyvinyl alcohol film outer surface.

22. A substrate, which is a wipe, cloth or sponge, for household use, or a water soluble household cleaning unit dose product, coated with at least one active containing material obtainable by introducing one or more gaseous or atomised liquid and/or solid coating-forming materials which undergo chemical bond forming reactions within a plasma environment and one or more active materials which substantially do not undergo chemical bond forming reactions within a plasma environment, into an atmospheric or low pressure plasma discharge and/or an excited gas stream resulting therefrom, and exposing the substrate to the resulting plasma treated mixture of atomised coating-forming and active materials.

23. A substrate, which is a wipe, cloth or sponge, for household or personal care, or a water soluble household cleaning unit dose product, coated with the product of a material formed by introducing one or more gaseous or atomised liquid and/or solid coating-forming materials which undergo chemical bond forming reactions within a plasma environment and one or more active materials which substantially do not undergo chemical bond forming reactions within a plasma environment, into an atmospheric or low pressure plasma discharge and/or an excited gas stream resulting therefrom.

24. A substrate according to claim 22 or 23, obtainable by a process as defined in any one of claims 1 to 21.

25. A product comprising a substrate as defined in any one of claims 22 to 24.

## Patentansprüche

1. Verfahren zum Bilden einer wirkstoffhaltigen Beschichtung auf einem Substrat, wobei es sich bei dem Substrat um ein Wischtuch, einen Lappen oder einen Schwamm für die Verwendung im Haushalt oder ein wasserlösliches Einheitsdosisprodukt für die Reinigung im Haushalt handelt, wobei das Verfahren die folgenden Schritte umfasst:
i) Einbringen von einem oder mehreren gashaltigen oder zerstäubten flüssigen und/oder festen beschichtungsbildenden Stoffen die sich einer Reaktionen in einer Plasmaumgebung bildenden chemischen Bindung unterziehen, und von einem oder mehreren Wirkstoffen, die sich im Wesentlichen keinen Reaktionen in einer Plasmaumgebung bildenden chemischen Bindung unterziehen, in eine atmosphärische oder Niederdruckplasmaentladung und/oder einen daraus erhaltenen angeregten Gasstrom, und
ii) Aussetzen des Substrats dem erhaltenen Gemisch aus einem zerstäubten beschichtungsbildenden Stoff und mindestens einem Wirkstoff, die auf der Substratoberfläche unter Bildung einer Beschichtung abgeschieden werden.

2. Verfahren nach Anspruch 1, wobei der beschichtungsbildende Stoff mithilfe von einem oder mehreren Zerstäubern in die Plasmaentladung eingebracht wird.

3. Verfahren nach Anspruch 2, **dadurch gekennzeichnet, dass** jeder Zerstäuber eine Ultraschalldüse ist.

4. Verfahren nach einem der vorangehenden Ansprüche, wobei der Wirkstoff durch denselben Zerstäuber wie der beschichtungsbildende Stoff in die Plasmaentladung eingebracht wird.

5. Verfahren nach einem der Ansprüche 1 bis 3, wobei der Wirkstoff mithilfe eines separaten Mittels zur Einbringung von Wirkstoffen) in die Plasmaentladung eingebracht wird.

6. Verfahren nach Anspruch 5, wobei das Mittel zur Einbringung von Wirkstoffen ein Zerstäuber oder im Falle von Pulvern eine Pulverspeiseeinrichtung mithilfe von komprimiertem Gas oder Schwerkraft ist.

7. Verfahren nach einem der vorangehenden Ansprüche, wobei das Substrat durch das Plasma und/oder den daraus erhaltenen angeregten Gasstrom geleitet wird.

8. Verfahren nach einem der vorangehenden Ansprüche, wobei die Behandlung der Substratoberfläche (1) entfernt von dem Plasmaentladungsplasma und/oder dem daraus erhaltenen angeregten Gasstrom durchgeführt wird.

9. Verfahren nach einem der vorangehenden Ansprüche, wobei der Wirkstoff ein oder mehrere Antimikrobizide, Enzyme, Proteine, Aloe und Vitamine, Duftstoffe und/oder Katalysator umfasst.

10. Verfahren nach einem der vorangehenden Ansprüche, wobei der Wirkstoff ein pharmazeutischer Stoff oder ein kosmetisch, therapeutischer oder diagnostischer Wirkstoff ist.

11. Verfahren nach einem der vorangehenden Ansprüche, wobei der Wirkstoff ein Antiseptikum, Antimykotikum, Antibakterizid, Antimikrobizid, Biozid, proteolytisches Enzym oder Peptid ist.

12. Verfahren nach einem der Ansprüche 1 bis 10, wobei der Wirkstoff ein UV-Screening-Stoff, ein Antioxidans, ein Flammschutzmittel, ein Antibakterizid, ein Antimykotikum, ein Reinigungsmittel, Aloe, ein Vitamin, ein Duftstoff oder ein Katalysator ist.

13. Verfahren nach einem der Ansprüche 1 bis 10, wobei der Wirkstoff ein Absorptionsmittel, ein Antioxidans, ein Antistatikum, ein Bindemittel, ein Pufferstoff, ein Strukturstoff, ein Chelatbildner, ein Farbmittel, ein deodorierender Stoff, ein Weichmacher, ein externes Analgetikum, ein Filmbildner, ein Duftstoffzusatz, ein Feuchtmittel, ein feuchtigkeitsspendender Stoff, ein Trübungsstoff, ein oxidierender oder reduzierender Stoff, ein Durchdringungsverbesserer, ein Weichmacher, ein Konservierungsmittel, ein Hautconditioner, ein Gleitmodifikator, ein löslichmachender Stoff, ein Lösungsmittel, ein Oberflächenmodifikator, ein oberflächenaktiver oder emulgierender Stoff, ein suspendierender Stoff, ein Verdickungsstoff, ein viskositätsregulierender Stoff, einschließlich eines viskositätserhöhenden oder -senkenden Stoffs oder ein UV-Licht-Absorptionsmittel ist.

14. Verfahren nach einem der Ansprüche 1 bis 10, wobei der Wirkstoff ein Pestizid- und/oder Fungizidwirkstoff ist.

15. Verfahren nach einem der vorangehenden Ansprüche, wobei das Substrat plasmavorbehandelt und/oder -nachbehandelt ist.

16. Verfahren nach Anspruch 15, wobei die Plasmanachbehandlung die Aufbringung einer zusätzlichen wirkstofffreien Beschichtung als Deckschicht umfasst.

17. Verfahren nach einem der vorangehenden Ansprüche, wobei eine Mehrzahl von einen oder mehrere Wirkstoffe enthaltenden Beschichtungen auf das Substrat aufgebracht wird.

18. Verfahren nach einem der vorangehenden Ansprüche, wobei die Beschichtung mithilfe einer plasmaverstärkten chemischen Aufdampfung aufgebracht wird.

19. Verfahren nach einem der vorangehenden Ansprüche, wobei das Substrat ein Textilmaterial für die Reinigung von harten Oberflächen ist.

20. Verfahren nach einem der Ansprüche 1 bis 18, wobei das Substrat ein Schwamm für die Reinigung von harten Oberflächen ist.

21. Verfahren nach einem der Ansprüche 1 bis 18, wobei das Substrat ein wasserlösliches Einheitsdosisprodukt für die Reinigung im Haushalt ist, das eine Außenfläche aus einer Polyvinylalkoholfolie umfasst.

22. Substrat, bei welchem es sich um ein Wischtuch, einen Lappen oder einen Schwamm handelt, zur Verwendung im Haushalt, oder ein wasserlösliches Einheitsdosisprodukt für die Reinigung im Haushalt, beschichtet mit mindestens einem wirkstoffhaltigen Material, erhältlich durch Einbringen von einem oder mehreren gashaltigen oder zerstäubten flüssigen und/oder festen beschichtungsbildenden Stoffen die sich einer Reaktionen in einer Plasmaumgebung bildenden chemischen Bindung unterziehen, und von einem oder mehreren Wirkstoffen, die sich im Wesentlichen keinen Reaktionen in einer Plasmaumgebung bildenden chemischen Bindung unterziehen, in eine atmosphärische oder Niederdruckplasmaentladung und/oder einen daraus erhaltenen angeregten Gasstrom, und Aussetzen des Substrats dem erhaltenen plasmabehandelten Gemisch aus zerstäubten beschichtungsbildenden Stoffen und Wirkstoffen.

23. Substrat, bei welchem es sich um ein Wischtuch, einen Lappen oder einen Schwamm handelt, zur Verwendung im Haushalt oder bei der Körperpflege, oder ein wasserlösliches Einheitsdosisprodukt für die Reinigung im Haushalt, beschichtet dem Produkt eines Stoffs, gebildet durch Einbringen von einem oder mehreren gashaltigen oder zerstäubten flüssigen und/oder festen beschichtungsbildenden Stoffen die sich einer Reaktionen in einer Plasmaumgebung bildenden chemischen Bindung unterziehen, und von einem oder mehreren Wirkstoffen, die sich im Wesentlichen keinen Reaktionen in einer Plasmaumgebung bildenden chemischen Bindung unterziehen, in eine atmosphärische oder Niederdruckplasmaentladung und/oder einen daraus erhaltenen angeregten Gasstrom.

24. Substrat nach Anspruch 22 oder 23, das durch ein wie in einem der Ansprüche 1 bis 21 definiertes Verfahren erhältlich ist.

25. Produkt, umfassend ein wie in einem der Ansprüche 22 bis 24 definiertes Substrat.

## Revendications

1. Procédé de formation d'un revêtement contenant une matière active sur un substrat, ledit substrat étant une lingette, un morceau de textile ou une éponge à usage domestique, ou un produit de nettoyage domestique soluble dans l'eau sous forme de dose unitaire, ledit procédé comprenant les étapes consistant à :
i) introduire une ou plusieurs matières liquides et/ou solides, gazeuses ou atomisées, formant un revêtement, qui subissent des réactions formant des liaisons chimiques dans un environnement de plasma, et une ou plusieurs matières actives qui ne subissent sensiblement pas de réactions formant des liaisons chimiques dans un environnement de plasma, dans une décharge de plasma à la pression atmosphérique ou à basse pression et/ou dans un flux gazeux excité qui en résulte, et
ii) exposer le substrat au mélange résultant de la matière atomisée formant un revêtement et d' au moins une matière active qui sont déposées à la surface du substrat pour former un revêtement.

2. Procédé selon la revendication 1, dans lequel la matière formant un revêtement est introduite dans la décharge de plasma au moyen d'un ou plusieurs atomiseurs.

3. Procédé selon la revendication 2, **caractérisé en ce que** chaque atomiseur est une buse à ultrasons.

4. Procédé selon l'une quelconque des revendications précédentes, dans lequel la matière active est introduite dans la décharge de plasma au moyen du même atomiseur que la matière formant un revêtement.

5. Procédé selon l'une quelconque des revendications 1 à 3, dans lequel la matière active est introduite dans la décharge de plasma par un moyen séparé d'introduction de matière active.

6. Procédé selon la revendication 5, dans lequel le moyen d'introduction de matière active est un atomiseur ou, dans le cas de poudres, est un dispositif d'amenée de poudre au moyen d'un gaz comprimé ou par gravité.

7. Procédé selon l'une quelconque des revendications précédentes, dans lequel on fait passer le substrat à travers le plasma et/ou le flux gazeux excité qui en résulte.

8. Procédé selon l'une quelconque des revendications précédentes, dans lequel on effectue le traitement de la surface du substrat (1) à distance de la décharge de plasma et/ou du flux gazeux excité qui en résulte.

9. Procédé selon l'une quelconque des revendications précédentes, dans lequel la matière active comprend un ou plusieurs éléments choisis parmi des agents antimicrobiens, enzymes, protéines, aloès et vitamines, parfums et/ou catalyseur.

10. Procédé selon l'une quelconque des revendications précédentes, dans lequel la matière active est une matière pharmaceutique ou une matière active du point de vue cosméceutique, une matière active du point de vue thérapeutique ou une matière active du point de vue diagnostique.

11. Procédé selon l'une quelconque des revendications précédentes, dans lequel la matière active est un agent antiseptique, un agent antifongique, un agent antibactérien, un agent antimicrobien, un biocide, une enzyme protéolytique ou un peptide.

12. Procédé selon l'une quelconque des revendications 1 à 10, dans lequel la matière active est une matière filtrant les rayons UV, un antioxydant, un agent ignifugeant, un agent antibactérien, un agent antifongique, un agent de nettoyage, l'aloès, une vitamine, un parfum ou un catalyseur.

13. Procédé selon l'une quelconque des revendications 1 à 10, dans lequel la matière active est un absorbant, un antioxydant, une matière antistatique, un liant, une matière de tamponnage, une matière de charge, une matière de chélation, un colorant, une matière désodorisante, un émollient, un analgésique externe, un agent filmogène, un ingrédient de parfum, un humidifiant, une matière hydratante, une matière opacifiante, une matière oxydante ou réductrice, une matière stimulant la pénétration, un plastifiant, un agent de conservation, une matière de conditionnement de la peau, un modificateur de glissement, une matière solubilisante, un solvant, un modificateur de surface, un agent de surface ou une matière émulsionnante, une matière de mise en suspension, une matière épaississante, une matière maîtrisant la viscosité, y compris une matière augmentant ou diminuant la viscosité, ou une matière absorbant la lumière UV.

14. Procédé selon l'une quelconque des revendications 1 à 10, dans lequel la matière active est une matière active du point de vue pesticide et/ou fongicide.

15. Procédé selon l'une quelconque des revendications précédentes, dans lequel le substrat est prétraité et/ou post-traité avec un plasma.

16. Procédé selon la revendication 15, dans lequel le post-traitement avec un plasma comprend l'application d'un revêtement supplémentaire exempt de matière active en tant que couche supérieure.

17. Procédé selon l'une quelconque des revendications précédentes, dans lequel on applique une pluralité de revêtements contenant une ou plusieurs matières actives sur le substrat.

18. Procédé selon l'une quelconque des revendications précédentes, dans lequel on applique le revêtement au moyen d'un dépôt chimique en phase vapeur renforcé avec un plasma.

19. Procédé selon l'une quelconque des revendications précédentes, dans lequel le substrat est une matière textile pour le nettoyage de surfaces dures.

20. Procédé selon l'une quelconque des revendications 1 à 18, dans lequel le substrat est une éponge pour le nettoyage de surfaces dures.

21. Procédé selon l'une quelconque des revendications 1 à 18, dans lequel le substrat est un produit de nettoyage domestique soluble dans l'eau sous forme de dose unitaire comprenant une surface externe à base d'un film d'alcool polyvinylique.

22. Substrat, qui est une lingette, un morceau de textile ou une éponge, à usage domestique, ou un produit de nettoyage domestique soluble dans l'eau sous forme de dose unitaire, revêtu d'au moins une matière contenant une matière active pouvant être obtenue par introduction d'une ou plusieurs matières liquides et/ou solides, gazeuses ou atomisées, formant un revêtement, qui subissent des réactions formant des liaisons chimiques dans un environnement de plasma et d'une ou plusieurs matières actives qui ne subissent sensiblement pas de réactions formant des liaisons chimiques dans un environnement de plasma, dans une décharge de plasma à la pression atmosphérique ou à basse pression et/ou dans un flux gazeux excité qui en résulte, et par exposition du substrat au mélange résultant, traité avec un plasma, de matières atomisées formant un revêtement et de matières actives.

23. Substrat, qui est une lingette, un morceau de textile ou une éponge, à usage domestique ou pour les soins corporels, ou un produit de nettoyage domestique soluble dans l'eau sous forme de dose unitaire, revêtu du produit d'une matière formée par introduction d'une ou plusieurs matières liquides et/ou solides, gazeuses ou atomisées, formant un revêtement, qui subissent des réactions formant des liaisons chimiques dans un environnement de plasma et d'une ou plusieurs matières actives qui ne subissent sensiblement pas de réactions formant des liaisons chimiques dans un environnement de plasma, dans une décharge de plasma à la pression atmosphérique ou à basse pression et/ou dans un flux gazeux excité qui en résulte.

24. Substrat selon la revendication 22 ou 23, pouvant être obtenu au moyen d'un procédé tel que défini dans l'une quelconque des revendications 1 à 21.

25. Produit comprenant un substrat tel que défini dans l'une quelconque des revendications 22 à 24.
